# EUROPÄISCHE PATENTANMELDUNG

(11) **EP 1 266 634 A1**
(43) Veröffentlichungstag der Anmeldung: **18.12.2002**
(21) Anmeldenummer: 02012836.9
(22) Anmeldetag: 10.06.2002
(51) Int. Cl.: A61C 13/30, A61K 6/08

(54) **Verfahren zum Behandeln eines Zahnes durch Einsetzen eines bruchfesten Stiftes in den Wurzelkanal**

(30) Priorität: 09.06.2001 DE 10128127
(71) Anmelder: Fischer-Brandies, Arndt, Dr., 87549 Rettenberg (DE); Deister, Franz, Dr., 81539 München (DE)
(72) Erfinder: Fischer-Brandies, Arndt, Dr., 87549 Rettenberg (DE); Deister, Franz, Dr., 81539 München (DE)
(74) Vertreter: Dosterschill, Peter, Dr.

(57) **Zusammenfassung**

Die Erfindung betrifft ein Verfahren zum Behandeln eines Zahnes, wobei in den Wurzelkanal (M) ein oder mehrere bruchfeste schlanke Stifte (S, So) eingesetzt werden, die zur Erhöhung der Zug- und Biegefestigkeit Fasern enthalten und die sich bis in den Bereich der Krone (So) hinein erstrecken, wobei die Stifte (S, So) mittels eines zwischen Stift (S) und Dentin (D) des Wurzelkanals eingebrachten, aushärtenden Befestigungsmittels (M) im Wurzelkanal befestigt werden. Ohne vorab den Wurzelkanal (M) durch künstliches Erweitern formschlüssig-genau an die Form der Stifte (S) anzupassen, werden die Stifte (S), die massiv in dem Sinne sind, dass sie längs ihrer Längsachse keine Kanüle oder Bohrung aufweisen, sofort in den nicht, oder höchstens teilweise, künstlich erweiterten, damit weiterhin natürliche Ausbuchtungen aufweisenden Wurzelkanal (M) eingesetzt werden.

## Beschreibung

Die Erfindung betrifft ein Verfahren zum Behandeln eines Zahnes nach dem Oberbegriff des Anspruchs 1, mit dem avitale Zähne wiederhergestellt werden.

Wenn man einen avitalen Zahn erhalten will, kann man ihn mit einem Wurzelstift - hier "Stift" genannt - ausstatten. Man versteift damit mehr oder weniger die Zahnwurzel und erschwert oder verhindert einen späteren Bruch. Soll bei einem solchen Zahn auch ein zerstörter anatomischer Kronenanteil ersetzt werden, muß der Stift noch einen Aufbau für eine künstliche Krone erhalten. Das erfindungsgemäße Verfahren eignet sich dabei sowohl für Behandlungen, bei denen der Zahn zusätzlich einen Aufbau für eine künstliche Zahnkrone (= "Krone") erhält, als auch für Behandlungen, bei denen der Zahn keinen solchen Aufbau erhält.

Oft erfolgt eine Vorbehandlung des Zahnes vor seiner Ausstattung mit einem Stift. Hierbei wird z.B. eine eventuell zuvor im Wurzelkanal angebrachte Wurzelfüllung entfernt, wobei auch der Wurzelkanal durch eine sog. Konditionierung gereinigt und eventuell Pulpabestandteile beseitigt werden können. Eventuell wird der besonders dünne letzte, nahe der Zahnwurzelspitze gelegene Abschnitt des Wurzelkanals (im Folgenden "Endabschnitt" genannt) im Rahmen dieser Vorbehandlung auch noch apikal verschlossen, bevor die Ausstattung des restlichen Wurzelkanals mit dem Stift beginnt. Die Erfindung betrifft also ein Verfahren zum Behandeln eines Zahnes, das im allgemeinen erst nach dem Ende einer solchen eventuellen Vorbehandlung beginnt.

Es gibt Verfahren zum Behandeln eines Zahnes, bei dem eine Besonderheit darin besteht, dass in den Wurzelkanal ein von der Zahnwurzel bis in den Bereich der Krone reichender schlanker Stift eingesetzt wird, der aus faserverstärktem Kunststoff besteht oder Kohlefasern enthält. Solche Fasern - damit auch der Stift - sind weitgehend bruchfest, d.h. sie besitzen eine besonders hohe Zugund Biegefestigkeit. Diese bekannten Stifte werden im "konfektionierten" Zustand im Handel angeboten, d.h. in verschiedenen Größen bzw. Stärken mit vom Stifthersteller vorgegebenen speziellen Stiftformen. Diese Stifte setzen voraus, dass - vor dem Einsetzen dieses Stiftes in den Wurzelkanal - ein passend präpariertes "Stiftbett" hergestellt wird: dass nämlich die Oberfläche des Dentins im Wurzelkanal und damit die Wurzelkanalform durch Ausbohren an die vorgegebene Form des Stiftes erst präzise formschlüssig angepasst wird. Das geschieht durch entsprechendes Erweitern (Ausbohren) des Wurzelkanals. Trotz dieser präzisen Anpassung der Form des Stiftbettes an die Form des Stiftes wird der Stift zusätzlich mittels eines aushärtenden Befestigungsmittels am Dentin des Stiftbettes befestigt. Als Befestigungsmittel werden Zement oder spezielle Kunststoffe benutzt.

Der Oberbegriff des Patentanspruches 1 bezieht sich auf ein spezielles derartiges Verfahren zum Behandeln eines Zahnes, das für sich durch die DE 199 57 857 A1, insbesondere durch deren Beschreibung in Spalte 1, Zeile 65 bis Spalte 2, Zeile 48, bekannt ist. Es handelt sich dort - wie bei der Erfindung - um ein Verfahren zum Behandeln eines Zahnes, bei dem in den Wurzelkanal ein von der Zahnwurzel bis in den Bereich der Krone reichender bruchfester schlanker Stift eingesetzt wird, der zur Erhöhung seiner Zug- und Biegefestigkeit Fasern enthält, indem er insbesondere aus faserverstärktem Kunststoff bestehen kann. Dieser Stift wird - ohne vorab den Wurzelkanal durch künstliches Erweitern formschlüssig-genau an die Form des Stiftes anzupassen - sofort in den Wurzelkanal eingesetzt, der seinerseits nicht - oder höchstens teilweise - künstlich erweitert wurde, weswegen der Wurzelkanal hierbei weiterhin natürliche Ausbuchtungen aufweist. Der Stift wird hierbei mittels eines zwischen dem Stift und dem Dentin des Wurzelkanals eingebrachten, aushärtenden Befestigungsmittels im Wurzelkanal befestigt.

Bei diesem durch die DE 199 57 857 A1 bekannten Verfahren besteht aber - abweichend von der Erfindung - eine Besonderheit darin, dass der eingesetzte schlanke Stift seinerseits eine aus faserverstärktem Kunststoff bestehende Kanüle darstellt, durch die das danach aushärtende Befestigungsmittel injiziert wird - wobei das Befestigungsmittel seinerseits insbesondere ein Kunststoff sein kann, der ebenfalls verstärkende Fasern enthält. Diese Kanüle soll schließlich im Zahn als verlorene Kanüle zurückbleiben und die Festigkeit des in den Wurzelkanal injizierten Befestigungsmittels nach dessen Aushärtung unterstützen. Die Erfindung vermeidet aber, den Stift durch eine längs der Längsachse des Stiftes liegende Kanüle darzustellen. Ein - besonders ein faserverstärkter - Kunststoff als Befestigungsmittel nämlich ziemlich zähflüssig, weswegen eine Kanüle als Stift, über die das Befestigungsmittel blasenfrei bis in die unterste Spitze des Wurzelkanals injiziert werden soll, einen ziemlich grossen Durchmesser aufweisen muss, um einen ausreichend niedrigen Durchflusswiderstand der Kanüle zu erreichen. Dieser grosse Kanülendurchmesser zwingt dazu, den vorhandenen Wurzelkanal /Nervkanal dafür entsprechend ausreichend bis in die unterste Spitze des Wurzelkanals zu erweitern. Die Erfindung soll auch diesen Zwang zur Erweiterung des Wurzelkanals / Nervkanals zumindest reduzieren, wenn nicht ganz vermeiden. Übrigens handelt es sich bei einer solchen Kanüle um einen konfektionierten Stift insofern, als bei ihm der Hersteller der Kanüle deren Form vorab festlegte.

Es gibt eine ganze Reihe weiterer bekannter Verfahren, bei denen ebenfalls konditionierte, von der Zahnwurzel bis in den Bereich der Krone reichende schlanke Stifte benutzt werden, die aber aus anderen Materialien bestehen. Sie bestehen z.B. aus Metall, aus Keramik oder aus Kunststoff, wobei auch die vom Stifthersteller vorgegebenen Formen dieser konfektionierten Stifte unterschiedlich sind, z.B. konisch, zylindrisch oder kombiniert zylindrisch-konisch. Bei allen diesen Verfahren ist dementsprechend durch Ausbohren ein konisches, zylindrisches oder zylindrisch-konisches Stiftbett herzustellen, wobei die Form des Stiftbettes jeweils präzise an die Form des konfektionierten Stiftes anzupassen ist und wobei auch dort die Stifte zusätzlich mittels des aushärtenden Befestigungsmittels am Dentin des Stiftbettes befestigt werden.

Ein Nachteil von allen bekannten Verfahren, die solche konfektionierten Stifte - oder auch Kanülen - zum Einsetzen in so präparierte Stiftbette benutzen, ist, dass durch das Herstellen des Stiftbettes - d.h. durch das Erweitern bzw. Ausbohren des Wurzelkanals - die Wandstärke des Dentins der Zahnwurzel jeweils deutlich verringert wird. Vgl. hierzu z.B. die DE 197 19 451 C1, in der u.a. beschrieben wird, dass mittels eines ebenfalls in der Zahnwurzel befestigten weiteren Sicherungsstiftes 26 das Verdrehen des angebrachten Kronenaufbaues verhindert werden soll. Die Erfindung vermeidet so starke Dentinverluste.

Verglichen mit diesen Verfahren, die solche konfektionierten Stifte benutzen, sind die hohen Dentinverluste bei sorgfältigem Vorgehen oft bereits mit einem weiteren bekannten Verfahren verringerbar. Bei diesem Verfahren werden nämlich - statt konfektionierter Stifte - individuell an die Form des Stiftbettes angepasste gegossene Metallstifte hoher Zug- und Biegefestigkeit benutzt, die ebenfalls von der Zahnwurzel bis in den Bereich der Krone hineinreichen. Bei diesem Verfahren wird während einer ersten Behandlungssitzung der Wurzelkanal zunächst vorsichtig durch Ausbohren - meistens konisch - erweitert sowie das so erzeugte Stiftbett geglättet und gereinigt. Danach wird ein Abdruck vom so präparierten Stiftbett hergestellt, um später im Labor den einzusetzenden Metallstift gießen zu können, wobei die Form des Stiftes individuell genau zur Form des so präparierten Stiftbettes passen soll. Am Ende dieser ersten Behandlungssitzung wird das Stiftbett provisorisch wieder verschlossen. Danach wird der Metallstift im Labor gegossen. Anschließend wird in einer folgenden, zweiten Behandlungssitzung das Stiftbett wieder geöffnet und gereinigt, wonach der gegossene Metallstift in das Stiftbett eingesetzt und dort mittels des Befestigungsmittels befestigt wird. Dieses bekannte Verfahren mit einem Metallstift, dessen Form individuell an die Form des präparierten Stiftbettes angepasst wird, ermöglicht oft, trotz der Wurzelkanalerweiterung besonders hohe Dentinverluste zu vermeiden, nämlich die Wandstärke des Dentins des Zahnwurzel im Vergleich zur Benutzung konfektionierter Stifte besser zu schonen. Dieses Verfahren erfordert jedoch einen besonders hohen Aufwand an Kosten, Zeit und Präzision - erfordert es doch (mindestens) zwei Behandlungssitzungen sowie solide Laborarbeiten. Überdies können Fehler nicht nur beim Anfertigen des Abdruckes des Stiftbettes auftreten, sondern auch beim Gießen des Stiftes, wobei auch die richtige Gießtemperatur einzuhalten ist.

Die Liste der mit der Erfindung gleichzeitig erreichbaren Vorteile ist ungewöhnlich lang und die Aufgabe der Erfindung ist entsprechend vielgestaltig. Die Erfindung ermöglicht nämlich, auf besondere Weise einen individuell an die Form des Wurzelkanals angepassten Stift benutzen zu können. Sie ermöglicht dabei,
- eine besonders hohe Zug- und Biegefestigkeit des Stiftes zu gewährleisten, obwohl vor dem Einbringen des Stiftes der Nervkanal / Wurzelkanal und damit in der Zahnwurzel die Dicke des Dentins nur äußerst schonend - wenn überhaupt - verringert wird,
- nicht nur - im Vergleich zu allen bekannten, ein Stiftbett für einen konfektionierten Stift benutzenden Verfahren - die hohen Dentinverluste deutlich zu vermindern,
- sondern auch - im Vergleich zu jenem bekannten Verfahren, bei dem bereits ein individuell an die Form des präparierten Stiftbettes angepasster Metallstift benutzt wird -,
   - den Aufwand an Zeit und Kosten sowohl für die zweite Behandlungssitzung als auch für das Anfertigen des Abdruckes und für die Laborarbeiten völlig einzusparen,
   - zusätzlich die Risiken beim Gießen des Stiftes im Labor und beim Anfertigen des Abdruckes völlig zu vermeiden,
   - zusätzlich die Dentinverluste besonders stark zu verringern,
   - und allergische Materialunverträglichkeiten des Patienten berücksichtigen zu können.
- Zudem ermöglicht die Erfindung, nicht nur einen stabilen Sitz des Stiftes zu erreichen, sondern auch bei Bedarf später einmal den Stift - insbesondere durch Ausbohren - unter Schonung der Wandstärke der Zahnwurzel leicht und weitgehend gefahrlos wieder zu beseitigen.
- Überdies eröffnet die Erfindung auch die Möglichkeit, einen Stift aus Materialien zu benutzen, die elastischer bleiben als Metallstifte, was vermeidet, dass die Zahnwurzel durch einen Metallstift zu stark versteift wird.
- Die Erfindung ermöglicht zudem, durch Einführen mehrerer Stifte in den Wurzelkanal den Stiftaufbau sofort und individuell an die bei der Behandlung vorgefundene Form des Wurzelkanals anzupassen, indem - insbesondere nacheinander - mehrere erfindungsgemäße Stifte nebeneinander in den Wurzelklanal eingeführt werden, wobei ein solches Stiftbündel seine endgültige, individuell an den behandelten Zahn angepasste Form und seine hohe Bruch- und Biegefestigkeit dentinschonend mittels des in den Wurzelkanal eingebrachten Befestigungsmittels erreicht; dies hat zur Folge, dass dann im wesentlichen die Form der Kanalwand die Form des Stiftbündels bestimmt.
- Die Erfindung ermöglicht zudem zumindest mitunter, schon mit einem einzigen erfindungsgemäß gestalteten Stift - statt mit einem ganzen Stiftbündel - auszukommen, insbesondere wenn die vorgefundene Dicke des Dentins der Zahnwurzel genügend stark und die vorgefundene Form des Wurzelkanals passend ist.

Diese Aufgabe der Erfindung wird durch das im Patentanspruch 1 genannte Verfahren gelöst.

Die in den Unteransprüchen definierten zusätzlichen Maßnahmen bzw. Umstände gestatten, jeweils zusätzliche Vorteile zu erreichen. Beispielsweise gestatten die Maßnahmen gemäß dem Patentanspruch
2, in ein und derselben Behandlungssitzung die resultierende Dicke des gesamten Bündels von Stiften sofort individuell an die jeweils vorgefundene Form des Wurzelkanals anzupassen, ohne den Wurzelkanal unbedingt zusätzlich erweitern zu müssen,
3 bis 6, eine Auswahl von Stiftmaterialien mit hoher Zug- und Biegefestigkeit und mit trotzdem relativ hoher Elastizität zu bieten, wobei nebenbei auch möglich wird, allergische Materialunverträglichkeiten von Patienten zu berücksichtigen,
7, schon das noch unausgehärtete Befestigungsmittel auszunutzen, um den Stift / die Stifte bis zur Aushärtung des Befestigungsmittels in der jeweils eingesetzten räumlichen Lage - oft ohne sonstige, den Stift / die Stifte festhaltende Hilfsmittel - zu stabilisieren, wobei überdies das Befestigungsmittel durch das folgende Einsetzen des Stiftes / der Stifte bis in Nischen innerhalb der Wände des Wurzelkanals gepreßt wird,
8, das Befestigungsmittel auch in die feinsten natürlichen Ausbuchtungen des Wurzelkanals besonders fest hineinzupressen, was die Bindung dieses Befestigungsmittels an das Dentin des Wurzelkanals und damit die Festigkeit der behandelten Zahnwurzel gegen Biege- und Druckkräfte beim späteren Kauen entsprechend verbessert,
9, die Möglichkeit zum Modellieren der Befestigungsmitteloberfläche zu verbessern,
10, eine besonders stabile Befestigung des Stiftes / der Stifte am Dentin der Zahnwurzel zu erreichen,
11, eine besonders stabile Befestigung des Stiftes / der Stifte am Befestigungsmittel zu erreichen, indem dann das Befestigungsmittelmaterial gut und unschwer an das Material der Stiftoberfläche angepasst werden kann,
12, die Bruchfestigkeit der Befestigungsmittels zu verbessern,
13, insbesondere durch die damit erreichbare Dentin-ähnliche Biegeelastizität des Befestigungsmittels möglichst zu vermeiden, dass beim Kauen - vor allem durch Biegen und Stauchen des Zahnes - Brüche innerhalb des Befestigungsmittels und / oder Risse an den Berührungsflächen zwischen dem Dentin des Wurzelkanals und dem Befestigungsmittel auftreten, selbst wenn der Stift, für sich alleine betrachtet, noch elastischer als das benutzte Befestigungsmittel ist,
14 und 15, mit wenig Aufwand einen Zahnstumpf aufbauen zu können,
sowie 16 bis 21, besonders geeignete Stifte und Befestigungsmittel zur Verfügung zu stellen.

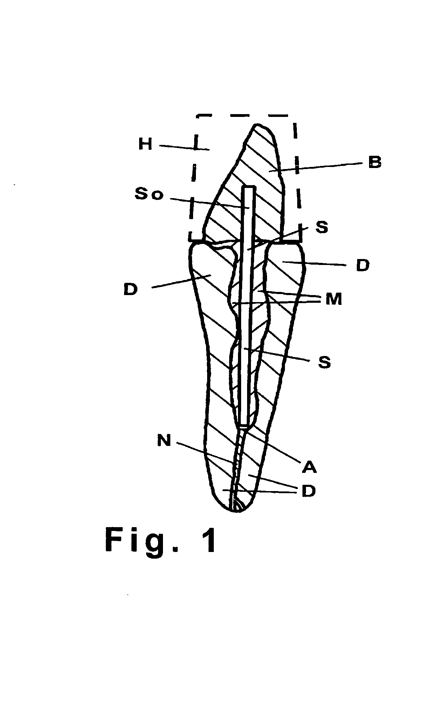

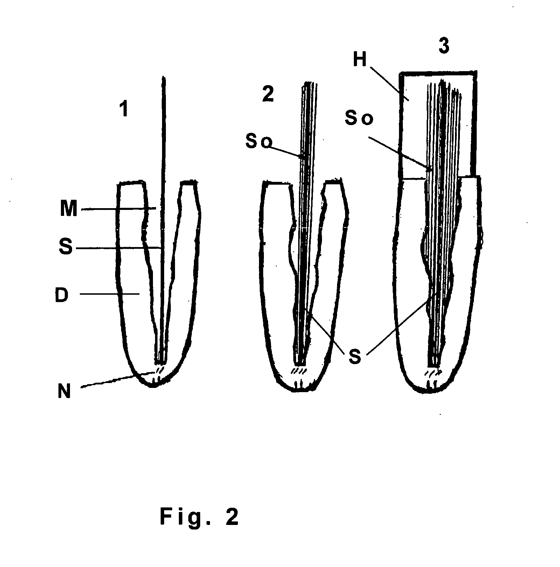

Die Erfindung und Weiterbildungen derselben werden nun anhand der in den beiden Figuren gezeigten Beispiele anschaulich erläutert. Dabei zeigt die Figur 1 ein Beispiel für einen mit einem einzelnen Stift behandelten Zahn. Die Figur 2 zeigt drei aufeinanderfolgende Behandlungsstufen eines gleichzeitig mit mehreren Stiften behandelten einzelnen Zahnes. Diese Figuren 1 und 2 zeigen dabei, um ihre Übersichtlichkeit zu verbessern, jeweils nur skizzenhaft die für die betreffende Erläuterung relevanten Details.

Es wird davon ausgegangen, dass bei Bedarf der Wurzelkanal M bereits von einer Wurzelfüllung befreit und durch Konditionierung gereinigt wurde, wobei auch Pulpabestandteile beseitigt und evtl. auch noch der Endabschnitt N bis zur Stelle A apikal verschlossen wurden. Danach beginnt die nun erläuterte erfindungsgemäße Behandlung.

Bei der Erfindung wird im Wurzelkanal M kein formschlüssig an die Stiftform angepasstes Stiftbett ausgebohrt, d.h. bei der Erfindung wird der Wurzelkanal M höchstens geringfügig künstlich etwas erweitert. Weil das künstliche Erweitern des Wurzelkanals M zumindest sehr weitgehend unterbleibt, weist der Wurzelkanal M bei der Erfindung weiterhin natürliche Ausbuchtungen bzw. große natürliche ungeglättete Abschnitte auf. Dadurch wird bei der Erfindung die Wandstärke des Dentins D der Zahnwurzel bestmöglich geschont und der Verlust an Dentin D durch Ausbohren minimiert oder ganz vermieden.

Erfindungsgemäß wird in den weiterhin natürliche Ausbuchtungen aufweisenden Wurzelkanal M der bruchfeste schlanke Stift S eingesetzt, der seinerseits zur Erhöhung seiner Zug- und Biegefestigkeit Fasern enthält. Mangels präziser Anpassung der Form des die natürlichen Ausbuchtungen aufweisenden Wurzelkanals an die Form des Stiftes des Wurzelkanals M kann der eingesetzte Stift S gar nicht - zumindest nicht überall - den Querschnitt des Wurzelkanals formschlüssig ausfüllen. Die Fasern des Stiftes S verlaufen im wesentlichen längs der Stiftachse - z.B. spiralig um die Stiftachse. Der Stift S erstreckt sich im Wurzelkanal M - bei dem in der Figur 1 gezeigten Beispiel in etwa von der Oberfläche des apikalen Verschlusses A an - bis in den Bereich So innerhalb der (natürlichen oder künstlichen) Krone, wobei nämlich bei der Erfindung die natürliche Krone des Zahnes noch vorhanden sein kann oder wobei erst nach der erfindungsgemäßen Behandlung noch eine künstliche Krone angebracht wird.

Zusätzlich wird das aushärtende Befestigungsmittel M in den Wurzelkanal M eingebracht, z.B. zusammen mit dem Stift S / So, oder vor dem Einsetzen dieses Stiftes S / So oder erst nach dem Einsetzen dieses Stiftes. Das in dieser Weise zwischen dem Stift S und dem Dentin D des Wurzelkanals eingebrachte, aushärtende Befestigungsmittel M befestigt den Stift S / So nach dem Aushärten im Wurzelkanal M. Im Unterschied zum Stand der Technik ist aber bei der Erfindung die Dicke des Befestigungsmittels M zwischen dem Stift S / So und dem Dentin D der Zahnwurzel ungleichmäßig dick und überdies keineswegs überall dünn.

Erfindungsgemäß ist der Stift S / So massiv, d.h. er besitzt längs seiner Längsachse keine eigens zum Injizieren des Befestigungsmittels M dienende Kanüle oder eine sonstige, zum Injizieren dieses Befestigungsmittels M dienende besondere Bohrung.

Weil die Erfindung das Herstellen des beim Stand der Technik üblichen Stiftbettes vermeidet, nämlich es unterlässt, zum Herstellen des Stiftbettes den Wurzelkanal M durch Ausbohren erheblich zu erweitern, ist bei der Erfindung der Dentinverlust besonders gering. Obwohl die Erfindung einen individuell an den zu behandelnden Zahn angepasste Behandlung bietet, vermeidet die Erfindung auch noch den hohen Aufwand an Zeit und Kosten für eine zweite Behandlungssitzung, auch den Aufwand an Zeit und Kosten für das Anfertigen eines Abdruckes und für die Laborarbeiten zum Gießen eines individuell an das besonders präparierte Stiftbett angepassten Metallstiftes. Damit vermeidet aber die Erfindung auch alle mit den Laborarbeiten und mit dem Anfertigen des Abdruckes verbundenen Risiken. Dabei gestattet die Erfindung unschwer, zur Berücksichtigung von allergischen Metallunverträglichkeiten des Patienten bestimmte Metalle zu vermeiden.

Zudem eröffnet die Erfindung unschwer die Möglichkeit, einen aus solchen Materialien hergestellten Stift zu benutzen, die elastischer bleiben als Metallstifte, was vermeidet, dass die Zahnwurzel durch einen Metallstift versteift wird.

Trotzdem wird bei der Erfindung nach dem Aushärten des Befestigungsmittels M ein sehr fester stabiler Sitz des Stiftes S im Wurzelkanal M erreicht, insbesondere weil sich das Befestigungsmittel M widerhakenartig an die natürlichen Ausbuchtungen des Wurzelkanals M anschmiegt. Überdies kann bei der Erfindung bei Bedarf später einmal der Stift - insbesondere durch Ausbohren - unter Schonung der Wandstärke der Zahnwurzel leicht und weitgehend gefahrlos wieder beseitigt, z.B. ausgebohrt, werden, wobei überdies auch nach diesem späteren Beseitigen bei schonendem Vorgehen die resultierenden Dentinverluste weiterhin gering bleiben.

Zusätzlich kann vor dem Aushärten des Befestigungsmittels M in den Spalt zwischen dem Dentin D des Wurzelkanals M und dem zunächst eingesetzten Stift S / So zumindest ein einziger weiterer - sogar mehrere weitere - schlanke zusätzliche ähnliche Stifte S / So im wesentlichen parallel zum zuerst eingesetzten Stift S / So eingesetzt werden. Die Figur 2 veranschaulicht drei aufeinanderfolgende Behandlungsstufen 1 bis 3 bei einer solchen Behandlung der Zahnwurzel. Gemäß der Figur 2 wird in der ersten Stufe 1 zunächst nur ein einziger Stift S / So - oder nur besonders wenige solche Stifte S / So - in den mit Befestigungsmittel M gefüllten Wurzelkanal eingeführt, insbesondere bis an die untere Spitze des Wurzelkanals M. Dabei muß der apikale Abschnitt N des Nervkanals / Wurzelkanals schon vor diesem Einführen dicht verschlossen worden sein.

Nach dem Einführen des ersten Stiftes S / So, bzw. nach dem Einführen von ersten Stiften S / So, werden gemäß der gezeigten Bahandlungsstufe 2 weitere Stifte S / So in den vorerst noch offenen oberen Abschnitt des Wurzelkanals M eingeführt, z.B. bis eine Füllung des Wurzelkanals M gemäß der gezeigten Behandlungsstufe 3 mehr oder weniger völlig mit erfindungsgemäßen Stiften aufgefüllt sind. Danach kann insbesondere mit Hilfe der sowohl in der Figur 1 als auch in der Figur 2 gezeigten Kappe H die Krone dieses Zahnes hergestellt werden. Gerade durch ein derartiges Auffüllen des Wurzelkanals M mit mehreren, nacheinander eingeführten Stiften S /So kann man nicht nur eine besonders grosse, dauerhaft stabile Zugund Biegefestigkeit des gesamten Stiftaufbaues erreichen. Dieser Aufbau gestattet darüber hinaus, ihn individuell an die momentan während der Behandlung vorgefundene Form des Wurzelkanals optimal anzupassen, ohne zuvor diesen Wurzelkanal durch entsprechendes Ausbohren unter Verlust von Dentin aufweiten zu müssen.

Diese zusätzlichen Stifte S / So stellen Verstärkungsstifte dar. Sie können jeweils aber auch dünner und / oder flacher als der zuerst eingesetzte Stift S / So sein. Dadurch kann z.B. der Querschnitt des Wurzelkanals M - wieder individuell dem momentan vorgefundenen Bedarf angepasst - auch mehr oder weniger prall mit Stiften S / So aufgefüllt werden. Diese Verstärkungsstifte gestatten demnach, die resultierende Dicke dieses gesamten Bündels von Stiften S / So sofort und unschwer unter Schonung der Wandstärke der Zahnwurzel individuell dem momentanen Bedarf anzupassen, insbesondere individuell an die vorgefundene Form des Wurzelkanalquerschnitts. Überdies gestattet so ein Bündel von Stiften S / So, schon in einer einzigen Behandlungssitzung eine besonders hohe Zug- und Bruchfestigkeit des behandelten Zahnes zu erreichen, ohne dazu den Wurzelkanal M erheblich erweitern zu müssen, besonders wenn elastisch biegbare Stifte benutzt werden.

Die Erfindung ist außerdem nicht an eine ganz spezielle Materialkombination für die Verbindung zwischen den Oberflächen des Stiftes S / So und des Befestigungsmittels M gebunden. Man kann nämlich z.B. Stifte S / So benutzen, die aus verschiedensten Materialien hergestellten Fasern besitzen, wobei ein Stift S / So im Prinzip gleichzeitig auch unterschiedliche Fasern aufweisen kann. So kann zumindest eine seiner Fasern eine Kohlefaser oder auch Glasfaser sein. Der Stift S / So kann auch einen faserverstärkten Kunststoff darstellen, wobei bei Bedarf durch Auswahl eines geeigneten Kunststoffes selbst allergische Kunststoffunverträglichkeiten von Patienten berücksichtigt werden können. Die Fasern können dabei jeweils streng parallel zur Stiftachse orientiert sein. Sie können aber auch ein Netz von vergitterten Fasern darstellen, die nur mehr oder weniger parallel zur Stiftachse orientiert sind. In allen diesen Fällen ist jeweils eine hohe Zug- und Biegefestigkeit sowie eine hohe Elastizität der Stifte S / So erreichbar.

Man kann das noch nicht unausgehärtete Befestigungsmittel M dazu mitausnutzen, um während des Aushärtens den Stift bzw. die Stifte in ihrer jeweils eingesetzten räumlichen Lage - in vielen Fällen ohne sonstige, den Stift / die Stifte festhaltende Hilfsmittel - zu stabilisieren. Dazu kann man zuerst das Befestigungsmittel M in den Wurzelkanal M einbringen, bevor der Stift / die Stifte in das mehr oder weniger klebrige, den Wurzelkanal M füllende Befestigungsmittel M eingesetzt werden. Wird der Stift / werden die Stifte erst nach dem Befestigungsmittel M in den Wurzelkanal M eingebracht, wird zusätzlich beim folgenden Einsetzen des Stiftes / der Stifte das Befestigungsmittel M an das Dentin D des Wurzelkanals angepreßt. Überdies kann das Befestigungsmittel M vor dem Aushärten zusätzlich unter Druck in den Wurzelkanal M gepresst werden, was die im Dentin D des Wurzelkanals M vorhandenen feinsten Ausbuchtungen - evtl. sogar bis in feinste seitliche Dentinkanäle - mit aushärtender Befestigungsmasse M auffüllt, was die widerhakenartige Bindung des Befestigungsmittels M an das Dentin D weiter verbessert. Man kann dabei auch die zur Krone hin gerichtete Oberfläche des Befestigungsmittels M nach Bedarf etwas modellieren, indem man einen Überschuß der Matrixmasse M vor ihrem völligen Aushärten - oder auch nach ihrem Aushärten - beseitigt.

Besondere zusätzliche Stifte, um nach Art der DE 197 19 451 C1 ein späteres Verdrehen der aufgesetzten Krone zuverlässig zu verhindern, ist bei der Erfindung nicht unbedingt nötig. Man kann nämlich den Aufbau B schließlich so formen - z.B. ihm durch Schleifen eine ovale Form geben, dass das Verdrehen der anschließend fertiggestellten Krone entsprechend zuverlässig verhindert wird.

Eine besonders stabile Befestigung des Stiftes, auch gleichzeitig mehrerer Stifte, am Dentin D der Zahnwurzel ist erreichbar, wenn das Befestigungsmittel M nach seinem Aushärten auch chemisch an den Oberflächen des Stiftes bzw. der Stifte und / oder an Oberflächen des Dentins D des Wurzelkanals haftet. Ein chemisches Haften ist besonders leicht erreichbar, wenn als Material für das Befestigungsmittel M dieselbe Masse, hier genannt "Matrixmasse", darstellt, die auch der faserhaltige Stift enthält, mit der also der Stift selber seine eigenen Fasern umhüllt. Faserhaltige Stifte enthalten nämlich eine Matrixmasse - im allgemeinen einen Kunststoff -, welche die eigenen Fasern der Stifte umhüllt bzw. einschließt. Die Stiftoberfläche besteht dann weitgehend aus dieser Matrixmasse des Stiftes.

Um die Bruchfestigkeit der Matrixmasse M weiter zu verbessern, kann die Matrixmasse M selbst eigene Fasern enthalten, die übrigens auch stochastisch orientiert sein können.

Wenn die Matrixmasse M nach ihrem Aushärten eine ähnliche Elastizität wie das in der Zahnwurzel enthaltene Dentin D aufweist, kann man dem Zerbrechen des Befestigungsmittels M beim Kauen durch Biegen und Stauchen des Dentins D entgegenwirken, selbst wenn dann der Stift S, für sich alleine betrachtet, noch elastischer als die Matrixmasse M ist.

Mit wenig Aufwand kann man anschließend auch den Aufbau eines Zahnstumpfes R herstellen, falls der Zahn zusätzlich mit einer künstlichen Krone auszustatten ist. Man kann dazu zunächst eine Kappe, z.B. aus Aluminium, ganz oder teilweise mit demselben Befestigungsmittel M füllen, das als Befestigungsmittel M im Wurzelkanal benutzt wurde. Anschließend stülpt man die gefüllte Kappe über jenes Ende So des Stiftes, das / über jene Enden So der Stifte, die aus dem in die Zahnwurzel eingebrachten Befestigungsmittel M herausragen - vgl. die in den Figuren 1 und 2 angedeutete Position H der über das Stiftende So gestülpten Kappe. Nach dem Entfernen der Kappe bilden zumindest Anteile des zuvor in der Kappe enthaltenen Befestigungsmittels M eine Art ring- oder hutartigen Körper über das aus der behandelten Zahnwurzel D herausragende Stiftende So / über die herausragenden Stiftenden So. Dieser Körper kann nach seinem Aushärten beschliffen werden und als Aufbau B dienen, auf dem danach die Krone angebracht wird.

Oben wurde bereits gezeigt, dass verschiedenste Stiftmaterialien und Befestigungsmittel für das erfindungsgemäße Verfahren geeignet sind. Der Stift kann Fasern hoher Zug- und Biegefestigkeit enthalten, z.B. Kohlefasern oder Glasfasern. Auch ein faserverstärkter Kunststoff kann benutzt werden. Die Matrixmasse M kann nach ihrem Aushärten insbesondere eine ähnliche Elastizität wie das in der Zahnwurzel enthaltene Dentin D aufweisen, wobei das Befestigungsmittel M selber zur Verbesserung seiner Bruchfestigkeit ebenfalls Fasern enthalten kann.

### Bezugszeichenliste

- A: Oberfläche des apikalen Verschlusses
- B: Zahnstumpf, beschliffener Aufbau / Körper
- D: Zahnwurzel, Dentin der Zahnwurzel
- H: Position der Kappe
- M: Wurzelkanal, Befestigungsmittel im Wurzelkanal, Matrixmasse im Wurzelkanal
- N: Endabschnitt = apikal verschlossener Abschnitt des Wurzelkanals
- S: Stift
- So: Ende des Stiftes im Bereich der Krone

## Patentansprüche

1. Verfahren zum Behandeln eines Zahnes, wobei in den Wurzelkanal (M) ein bruchfester schlanker Stift (S, So) eingesetzt wird, der zur Erhöhung seiner Zug- und Biegefestigkeit Fasern enthält und sich bis in den Bereich der Krone (So) hinein erstreckt, wobei der Stift (S, So) mittels eines zwischen dem Stift (S) und dem Dentin (D) des Wurzelkanals eingebrachten, aushärtenden Befestigungsmittels (M) im Wurzelkanal befestigt wird, und wobei - ohne vorab den Wurzelkanal (M) durch künstliches Erweitern formschlüssig-genau an die Form des Stiftes (S) anzupassen - der Stift (S) sofort in den nicht, oder höchstens teilweise, künstlich erweiterten, damit weiterhin natürliche Ausbuchtungen aufweisenden Wurzelkanal (M) eingesetzt wird, **dadurch gekennzeichnet, dass** ein Stift (S) benutzt wird, der massiv in dem Sinne ist, dass er längs seiner Längsachse keine zum Injizieren dienende Kanüle oder Bohrung aufweist.

2. Verfahren nach Patentanspruch 1, **dadurch gekennzeichnet ,dass** in den Spalt zwischen dem Dentin (D) des Wurzelkanals und dem Stift (S/So) im wesentlichen parallel zum Stift (S/So) - vor dem Aushärten des Befestigungsmittels (M) - zumindest ein einziger schlanker zusätzlicher gleichartiger Stift (S/So) als Verstärkungsstift eingefügt wird.

3. Verfahren nach Patentanspruch 1 oder 2, **dadurch gekennzeichnet, dass** der Stift (S) bzw. zumindest einer der verwendeten Stifte (S) zu einem Netz vergitterte Fasern enthält.

4. Verfahren nach einem der vorhergehenden Patentansprüche, **dadurch gekennzeichnet, dass** zumindest eine der Fasern von zumindest einem der verwendeten Stifte (S) eine Kohlefaser ist.

5. Verfahren nach einem der vorhergehenden Patentansprüche, **dadurch gekennzeichnet, dass** zumindest eine der Fasern von zumindest einem der verwendeten Stifte (S) eine Glasfaser ist.

6. Verfahren nach einem der vorhergehenden Patentansprüche, **dadurch gekennzeichnet, dass** zumindest einer der verwendeten Stifte (S) einen faserverstärkten Kunststoff darstellt.

7. Verfahren nach einem der vorhergehenden Patentansprüche, **dadurch gekennzeichnet, dass** zuerst das Befestigungsmittel (M) in den Wurzelkanal eingebracht wird, bevor der Stift (S) /die Stifte (S) in den Wurzelkanal eingebracht werden.

8. Verfahren nach einem der vorhergehenden Patentansprüche, **dadurch gekennzeichnet, dass** das Befestigungsmittel (M) vor seinem Aushärten unter Druck in den Wurzelkanal gepresst wird.

9. Verfahren nach einem der vorhergehenden Patentansprüche, **dadurch gekennzeichnet, dass** ein Überschuß des Befestigungsmittels beseitigt wird.

10. Verfahren nach einem der vorhergehenden Patentansprüche, **dadurch gekennzeichnet, dass** das Befestigungsmittel (M) nach seinem Aushärten auch chemisch an Oberflächen des Stiftes (S) / der Stifte (S) und / oder an Oberflächen des Dentins (D) des Wurzelkanals haftet.

11. Verfahren nach einem der vorhergehenden Patentansprüche, bevorzugt nach Patentanspruch 10, **dadurch gekennzeichnet,dass** als Befestigungsmittel (M) dieselbe Matrixmasse (M) benutzt wird, die auch der Stift (S / So) enthält und mit der (M) der Stift (S / So) seine eigenen Fasern umhüllt.

12. Verfahren nach einem der vorhergehenden Patentansprüche, **dadurch gekennzeichnet, dass** das Befestigungsmittel (M) eigene Fasern enthält.

13. Verfahren nach einem der vorhergehenden Patentansprüche, **dadurch gekennzeichnet, dass** das Befestigungsmittel (M) nach dem Aushärten eine ähnliche Elastizität wie das Dentin (D) der Zahnwurzel aufweist.

14. Verfahren zum Behandeln eines mit einer künstlichen Krone auszustattenden Zahnes nach einem der vorhergehenden Patentansprüche, **dadurch gekennzeichnet,**
- **dass** eine mit Befestigungsmittel (M) im wesentlichen gefüllte Kappe (H) vorübergehend über jenes Ende (So) des Stiftes (S) /der Stifte (S) gestülpt wird, das (So) aus dem in die Zahnwurzel (D) eingebrachten Befestigungsmittel (M) herausragt,
- und **dass** nach dem Entfernen der Kappe (H) zumindest Anteile des zuvor in der Kappe (H) enthaltenen Befestigungsmittels (M) einen aushärtenden oder ausgehärteten Aufbau / Körper bilden, die das aus der Zahnwurzel (D, S, M) herausragende freie Ende (So) des Stiftes (S) / der Stifte (S) umgeben,

15. Verfahren nach Patentanspruch 14, **dadurch gekennzeichnet,dass** nach dem Aushärten des Körpers letzterer außen beschliffen wird und danach als Aufbau (B) dient, über dem schließlich die Krone angebracht wird.

16. Stift (S, So) zur Verwendung in einem Verfahren nach einem der vorhergehenden Patentansprüche, **dadurch gekennzeichnet,dass** er eine oder mehrere Fasern hoher Zug- und Biegefestigkeit enthält.

17. Stift (S, So) nach Patentanspruch 16, **dadurch gekennzeichnet,dass** zumindest eine seiner Fasern eine Kohlefaser ist.

18. Stift (S, So) nach Patentanspruch 16 oder 17, **dadurch gekennzeichnet,dass** zumindest eine seiner Fasern eine Glasfaser ist.

19. Stift (S, So) nach einem der Patentansprüche 16 bis 18, **dadurch gekennzeichnet, dass** er aus faserverstärktem Kunststoff besteht.

20. Befestigungsmittel (M) zur Verwendung in einem Verfahren nach einem der Patentansprüche 7 bis 15, **dadurch gekennzeichnet,dass** es (M) nach dem Aushärten eine ähnliche Elastizität wie das Dentin (D) der Zahnwurzel besitzt.

21. Befestigungsmittel (M) nach Patentanspruch 20 oder zur Verwendung in einem Verfahren nach einem der Patentansprüche 7 bis 15, **dadurch gekennzeichnet, dass** es selbst eigene Fasern enthält.
